# EUROPEAN PATENT APPLICATION

(11) **EP 4 687 147 A1**
(43) Date of publication of application: **04.02.2026**
(21) Application number: 24306308.8
(22) Date of filing: 02.08.2024
(51) Int. Cl.: G16H 10/60, G06N 3/0455, G16H 50/20, G16H 50/70, G06N 3/08

(54) **MEDICAL DATABASE SEARCHING SYSTEM**

(71) Applicant: Koninklijke Philips N.V., 5656 AG Eindhoven (NL)
(72) Inventor: SHRESHTHA, Kumar, 5656 Eindhoven (NL); FACURY DE SOUZA, Luiz, 5656 Eindhoven (NL); KHELDOUNI, Amine, 5656 Eindhoven (NL); DUPONT, Rémi, 5656 Eindhoven (NL); CHOFFIN, Benoît, 5656 Eindhoven (NL); PEZZOTTI, Nicola, 5656 Eindhoven (NL)
(74) Representative: Philips Intellectual Property & Standards

(57) **Abstract**

The present disclosure provides concepts for searching a medical database comprising electrocardiogram (ECG) data. The method includes obtaining a database comprising a plurality of subject entries, each corresponding to a historic subject and comprising recorded metadata describing characteristics of the respective subject and a recorded vector embedding representing ECG data of the respective subject. A query vector embedding representing ECG data of a query subject is generated with an ECG encoder. The recorded metadata of the plurality of subject entries is compared with query metadata describing characteristics of the query subject, and the recorded vector embeddings are compared with the query vector embedding. One or more similar subject entries are identified based on a result of the comparison. Accordingly, the invention provides a searching means that is designed to take into account characteristics of subjects to identify subjects with similar cardiological conditions, thereby reducing a burden on a clinician whilst reducing a rate of misdiagnosis. For example, this may be particularly useful for triaging subjects.

## Description

### FIELD OF INVENTION

The present invention relates to systems and methods for searching medical databases containing subject data, and more particularly to searching and comparing electronic health records using vector embeddings of electrocardiogram (ECG) data.

### BACKGROUND

Electrocardiograms (ECGs) are widely used as a primary diagnostic tool for cardiovascular diseases due to their relative ease and speed of acquisition, cheap measurement devices, and ability to provide valuable insights into a subject's cardiac status. However, interpreting ECG data requires significant expertise and a deep understanding of cardiac electrophysiology. This complexity creates a challenge in leveraging ECGs to their full potential, especially in settings where expert cardiologists may not be readily available.

While automated diagnostic algorithms have been developed to assist in ECG interpretation, these systems often struggle to account for the myriad of factors that influence care decisions. A subject's age, comorbidities, drug allergies, and other individual characteristics play crucial roles in determining appropriate treatment pathways, yet integrating this information with ECG analysis remains a significant challenge.

Large medical centers typically maintain extensive databases of electronic health records (EHRs) containing valuable historical data on treatments and outcomes. However, traditional database systems are limited in their ability to efficiently compare, and match subjects based on complex, non-standard data types such as ECG signals, medical images, and unstructured clinical notes. This limitation hinders the potential for leveraging past experiences to inform current care decisions and improve treatment outcomes.

As healthcare moves towards more personalized approaches, there is a growing need for systems that can effectively integrate diverse subject data, including rich biomedical signals, to support clinical decision-making. Addressing this challenge could significantly enhance the quality of care, reduce misdiagnosis rates, and alleviate the burden on healthcare providers.

### SUMMARY OF INVENTION

The invention is defined by the claims.

According to an aspect of the present disclosure, a method for searching a medical database comprising electrocardiogram (ECG) data is provided. The method includes obtaining a database comprising a plurality of subject entries each corresponding to a historic subject, each subject entry comprising recorded metadata describing characteristics of the respective subject and a recorded vector embedding representing ECG data of the respective subject. The method further includes generating, with an ECG encoder, a query vector embedding representing ECG data of a query subject. The method also includes comparing the recorded metadata of the plurality of subject entries with query metadata describing characteristics of the query subject, and the recorded vector embeddings with the query vector embedding. Additionally, the method includes identifying one or more similar subject entries based on a result of the comparison.

Proposed concepts thus aim to provide schemes, solutions, concepts, designs, methods, and systems pertaining to searching a medical database comprising a plurality of subject entries each corresponding to a historic subject. More particularly, the invention enables the identification of similar subjects to a query subject amongst the plurality of subjects. In some cases, by providing these matched subject entries to a clinician, triaging of the query subject may be performed by analysis of the diagnostic and treatment pathways of the matched historic subjects.

It has been realized that whilst rich ECG data contains important details as to the condition of the subject, it is a complex (if not impossible) task to accurately match ECG data. In other words, matching subjects using ECG data provides deeper insights into best care pathways for the subject but is a costly exercise that is difficult to automate.

Accordingly, the invention proposes to compare the ECG data in the form of a vector embedding. A vector embedding captures deep features within the ECG data in a format that enables comparison with other vector embeddings in a computationally efficient manner. Indeed, recent developments in vector databases have enabled the storage and comparison of rich data formats using vector embeddings. Thus, the invention leverages this recent advance, and applies this to ECG data to enable the accurate identification of similar subjects, which may be used to better understand the condition and likely care pathway of a query subject.

Furthermore, the identification of similar subject entries is also achieved by comparison of the recorded metadata (i.e., subject characteristic information) of the plurality of subject entries with query metadata of the query subject. Thus, the invention is able to take into account a large range of factors that are needed for the accurate understanding of the condition of the query subject. Indeed, diagnostic algorithms that simply analyze ECG data often do not reach the correct conclusion due to the significant role subject characteristics play in the diagnosis and decision of the appropriate care pathway for the subject.

In other words, the invention proposes to match subjects based on both their metadata (e.g., subject demographic information, subject medical information, subject condition information, and subject context information) and ECG data associated with the subject in the form of a vector embedding. Thus, similar historical subjects to a query subject may be identified that are similar in terms of characteristics (e.g., similar co-morbidities, age, etc.) and that have also produced similar ECG data. This matching provides information as to likely diagnosis for the query subject due to information associated with the historical subject, as well as a possible care pathway.

The disclosed invention therefore offers several advantages. By representation of ECG data in the form of a vector embedding, comparison of this rich data from a query subject with a large database is enabled. Furthermore, by considering a range of characteristics of the subject, they ensure that the identified similar subjects are similar both in produced ECG data, and corresponding circumstances. For example, a historical subject that produced identical ECG data but in vastly different circumstances (e.g., suffered from many different co-morbidities), may be a less useful match than a subject that produced ECG data differing in only small ways but having almost identical characteristics (e.g., similar ages, medical histories, backgrounds, etc.). This approach may thus ensure the identification of similar subject entries that are particularly useful for identifying probable diagnosis and care pathways, in particular for fast and simple triage of the subject.

In some embodiments, the comparing of the metadata and the vector embeddings may comprise filtering the historic subject entries to identify candidate subject entries, the candidate subject entries comprising similar metadata to the metadata of the query subject and comparing the vector embeddings of the identified candidate subject entries and the query vector embedding. This feature allows for efficient narrowing down of potential matches before performing more computationally intensive vector comparisons.

The comparing of the vector embeddings may comprise processing, with a hierarchical navigable small world (HNSW) algorithm, each of the vector embeddings of the identified candidate subject entries and the query vector embedding. This feature enables fast and efficient similarity searches in high-dimensional spaces.

The comparing of the vector embeddings may comprise generating, for each vector embedding of the identified candidate subject entries, a similarity score indicating a degree of similarity between the query vector embedding and the respective vector embedding of the identified candidate subject entry, and the identifying of the one or more similar subject entries may be based on the generated similarity scores. This feature allows for quantitative ranking of similarity between subjects, thus providing more information that is relevant for a clinician assessing the produced results. In turn, this may reduce a cognitive burden on the clinician.

The filtering of the historic subject entries may be based on a predefined set of criteria defining matching characteristics between the metadata of the subject entry and the metadata of the query subject. This feature enables customizable and flexible filtering based on specific requirements. The predefined set of criteria may be based on user preferences indicating target matching characteristics, allowing for personalized and context-specific searches.

In some embodiments, the ECG encoder may be a neural network adapted to detect features of ECG data and convert the detected features into a vector. Specifically, the neural network may be a vision transformer. This feature enables sophisticated feature extraction and representation of ECG data.

The method may further comprise providing at least part of the recorded metadata, the ECG data, reported clinical findings and/or subject outcomes associated with the identified similar subject entries. This thus enhances the utility of the search results for clinical decision-making, providing relevant data associated with the identified similar subject entries.

The method may also include ranking the identified similar subject entries based on a degree of similarity between the respective recorded vector embeddings of the similar subject entries and the query vector embedding, and/or the respective recorded metadata of the similar subject entries and the query metadata. This feature facilitates prioritization of search results, reducing cognitive burden of a clinician.

In particular, the metadata may comprise subject demographic information, subject medical information, subject condition information, and subject context information. These parameters may allow for comprehensive subject matching, including all characteristics that may be relevant for identifying subjects with probable similar diagnosis and care pathways.

The vector embeddings may be a projection of the ECG data in high dimension space that encodes semantic meaning of the ECG data, enabling nuanced representation of ECG characteristics.

According to another aspect of the present disclosure, a computer program comprising computer program code means adapted, when said computer program is run on a computer, to implement methods described above is provided.

According to yet another aspect of the present disclosure, a system for searching a medical database is provided. The medical database comprises a plurality of subject entries each corresponding to a historic subject, each subject entry comprising recorded metadata describing characteristics of the respective subject and a recorded vector embedding representing ECG data of the respective subject. The system includes an ECG encoder configured to generate a query vector embedding representing ECG data of a query subject. The system also includes a database interface configured to compare the recorded metadata of the plurality of subject entries with query metadata describing characteristics of the query subject, and the recorded vector embeddings with the query vector embedding and identify one or more similar subject entries based on a result of the comparison.

Specifically, the ECG encoder may be a neural network adapted to detect features of ECG data and convert the detected features into a vector. This feature enables sophisticated processing and representation of ECG data within the system.

These and other aspects of the invention will be apparent from and elucidated with reference to the embodiment(s) described hereinafter.

### BRIEF DESCRIPTION OF FIGURES

For a better understanding of the invention, and to show more clearly how it may be carried into effect, reference will now be made, by way of example only, to the accompanying drawings, in which:
FIG. 1 illustrates a flowchart for a method for searching a medical database comprising ECG data is provided, according to aspects of the present disclosure;
FIG. 2 depicts a bar graph comparing ground truth abnormalities associated with the 100 nearest matched subjects to a query subject according to a demonstration of the present invention;
FIG. 3A-3C show density distribution graphs for parameter values of an entire subject dataset and the 100 nearest matched subjects in comparison with the parameter value of the query subject;
FIG. 4 depicts a block diagram of a system for searching a medical database comprising ECG data, in accordance with example embodiments; and
FIG. 5 is a simplified block diagram of a computer within which one or more parts of an embodiment may be employed.

### DETAILED DESCRIPTION

The invention will be described with reference to the Figures.

It should be understood that the Figures are merely schematic and are not drawn to scale. It should also be understood that the same reference numerals are used throughout the Figures to indicate the same or similar parts.

It should also be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. The mere fact that certain measures are recited in mutually different dependent claims does not indicate that a combination of these measures cannot be used to provide an advantage.

The present disclosure provides concepts for searching a medical database comprising electrocardiogram (ECG) data. The method includes obtaining a database comprising a plurality of subject entries, each corresponding to a historic subject and comprising recorded metadata describing characteristics of the respective subject and a recorded vector embedding representing ECG data of the respective subject. A query vector embedding representing ECG data of a query subject is generated with an ECG encoder. The recorded metadata of the plurality of subject entries is compared with query metadata describing characteristics of the query subject, and the recorded vector embeddings are compared with the query vector embedding. One or more similar subject entries are identified based on a result of the comparison. Accordingly, the invention provides a searching means that is designed to take into account characteristics of subjects to identify subjects with similar cardiological conditions, thereby reducing a burden on a clinician whilst reducing a rate of misdiagnosis. For example, this may be particularly useful for triaging subjects.

Accordingly, disclosed embodiments provide concepts for searching and comparing entries in a medical database, such as electronic health records, using vector embeddings of electrocardiogram (ECG) data. By leveraging advanced machine learning techniques and vector database technologies, the invention enables efficient and accurate identification of similar cases to support clinical decision-making.

A key aspect of the invention is the use of an ECG encoder, which may convert complex ECG signals into high-dimensional vector embeddings that capture the semantic meaning of the ECG data. This allows for nuanced comparisons between ECG signals that go beyond simple pattern matching, potentially revealing subtle similarities in cardiac conditions that may not be immediately apparent through traditional analysis methods.

The system combines these ECG vector embeddings with subject metadata to perform multi-faceted searches of large subject databases. By filtering candidate matches based on relevant metadata before performing potentially computationally intensive vector comparisons, the system achieves both accuracy and efficiency in identifying similar cases. The use of advanced algorithms, such as the Hierarchical Navigable Small World (HNSW) algorithm, further enhances the speed and effectiveness of similarity searches in high-dimensional spaces.

The invention offers several advantages over existing methods of ECG analysis and subject matching. It has the potential to reduce misdiagnosis rates by providing clinicians with relevant historical cases for comparison. The system can also aid in subject triage by quickly identifying potentially severe cases based on similarities to past subjects. Furthermore, by enabling healthcare providers to leverage their extensive databases of past subject outcomes, the invention supports evidence-based decision-making and moves towards more personalized subject care.

The present disclosure thus provides a health IT infrastructure that leverages vector databases of ECG embeddings, among other complex data modalities, to facilitate the matching of a query subject to multiple subjects, diagnostic and treatment histories. More particularly, subjects with similar physiological characteristics and with similar features in their ECGs to a query subject are identified, and as a result the matched subjects diagnostic and treatment histories are identified. These matched subject diagnostic and treatment histories may be used to triage and provide an estimated clinical pathway for the query subject. This infrastructure is thus designed to enhance the precision of medical care by enabling the comparison of a new subject's health information and ECG data with existing subject records in the database.

More specifically, the invention utilizes an ECG encoder, which may be a neural network or another machine learning model, to convert ECG data into vector embeddings. These embeddings, which represent the ECG data in a high-dimensional space, encode the semantic meaning of the signal, allowing for the comparison of physiological similarities between subjects. That is, the conversion of ECG data into vector embeddings enables meaningful and computational efficiency comparison of the ECG data, which otherwise is a highly complex and manual process often requiring the input of highly trained clinicians.

The invention incorporates a process for querying the vector database using both the subject's health information (i.e., subject metadata) and the ECG embedding. This process involves filtering subjects based on defined criteria and identifying those with ECG embeddings closest to the query subject. The identified subjects' Electronic Health Records (EHRs) are then presented to the treating clinician, providing valuable insights into potential treatment pathways based on historical data.

In some embodiments, the invention provides for ranking of the matched subjects based on their similarity score to the query subject and present their respective treatment pathways and clinical outcomes to the clinician. This feature may assist in clinical decision-making and potentially improve the triage process for subjects requiring immediate attention.

Furthermore, it is worth noting that while the system is described primarily in the context of ECG data, it may also be adapted to work with other types of clinical data, such as medical imaging, lab test reports, clinician notes, and any combination thereof. This flexibility allows for a more holistic approach to subject matching and clinical decision support, further enhancing the potential for precision medicine.

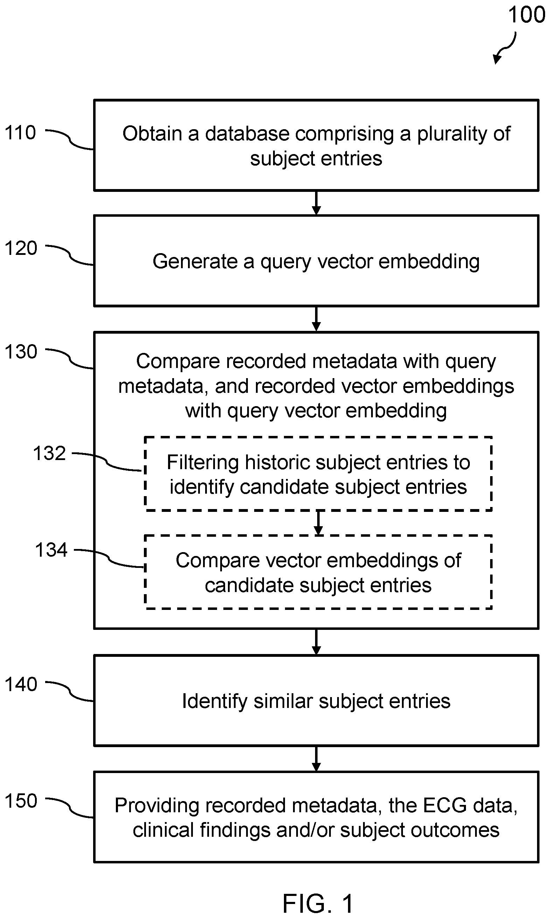

Referring to FIG. 1, the flowchart illustrates a method for identifying similar subject entries in a medical database. Specifically, the method may enable a user to start from ECG data and metadata (e.g., clinical information) of a query subject, and identify one or more diagnostic and treatment histories of subjects that are similar to the query subject. The medical database may include electronic medical records, electronic health records, or other databases that comprise ECGs associated with a subject. The aim of the method is to identify entries in the medical database associated with subjects that are similar to the query subject based on metadata of the subjects and ECG data associated with the subjects. Ultimately, this may enable a clinician to find outcomes and treatment for subjects in similar conditions to the query subject.

In step 110, a database comprising a plurality of subject entries is obtained. Each subject entry corresponds to a historic subject and includes recorded metadata describing characteristics of the respective subject and a recorded vector embedding representing ECG data of the respective subject. Obtaining the database may simply involve gaining access to the database so that at least part of the database may be searched.

The recorded metadata may include, in some cases, subject demographic information (e.g., age, race, sex), subject medical information (e.g., co-morbidities, condition history), subject condition information (e.g., present diagnosis, present ailments, present rest level at time of ECG data acquisition), and subject context information (e.g., environmental conditions and stress factors during acquisition of ECG signal). Essentially, the metadata indicates or describes characteristics of the associated subject that may be relevant to interpretation of an associated ECG recording, a diagnosis, a treatment, or a care pathway for the subject.

The vector embeddings are a projection of the ECG data in high dimensional space that encodes the semantic meaning of the ECG data. Put another way, vector embeddings capture deep features within the data that may not be immediately apparent upon inspection of the raw ECG data. Therefore, vector embeddings that are close together in this space indicate that the ECG data represented by the vector embeddings are physiologically closer than the vector embeddings that are far apart in this space.

In step 120 a query vector embedding is generated. Similarly to the above, the query vector embedding is a projection of ECG data of the query subject in high dimensional space that encodes semantic meaning of the ECG data. This is accomplished with an ECG encoder, which may perform any function for generating a vector embedding from ECG data that then captures features of the data in such a way that similar sets of ECG data are represented by vector embeddings that are close together in space. That is, the ECG encoder generates a query vector embedding representing ECG data of a query subject.

More specifically, the ECG encoder may be a neural network or another machine learning model, adapted to detect features of ECG data and convert the detected features into a vector. In other words, the ECG encoder is trained to extract and summarize signal characteristics from ECG data.

In some embodiments, the neural network may be a vision transformer that is trained in a self-supervised manner to detect features on an ECG such that the resulting vector embedding is close for ECGs that are similar while far apart for the ones that are dissimilar.

In step 130, the recorded metadata of the plurality of subject entries is compared with query metadata describing characteristics of the query subject, and the recorded vector embeddings are compared with the query vector embedding. The comparison may involve determining a difference between each of the subject entries and the query subject, and thus identifying which subject entries are most similar to the query subject.

More particularly, this comparison step may include two sub-steps: filtering 132 historic subject entries to identify candidate subject entries and comparing 134 vector embeddings of candidate subject entries.

In step 132, the historic subject entries are filtered to identify candidate subject entries, with the candidate subject entries comprising similar metadata to the metadata of the query subject. That is, any historic subjects having metadata that closely matches or closely resembles that of the query subject are identified as potential/candidate subject entries. For example, if the metadata indicates similar ages (e.g., +- 5 years), co-morbidities (e.g., both have similar blood pressure issues), and subject conditions (e.g., both underweight), then the historic subject entry may be identified as a candidate subject entry. This identification of candidate subject entries may, in a computational quick and efficient manner, reduce the number of vector embeddings that need to be compared to identify closest matching subject entries in the database.

In some instances, some parts of the metadata may need to match perfectly, other parts of the metadata may only need to be similar, and some parts of the metadata may only need to match optionally. This may relate to how important/relevant those characteristics described by the metadata are to the cardiovascular condition of a subject.

More specifically, the filtering 132 of the historic subject entries may be based on a predefined set of criteria defining matching characteristics between the metadata of the subject entry and the metadata of the query subject. Thus, it may be provided that, for a historic subject entry to be considered a candidate subject entry, then perfect matches in some parts of the metadata may be required, with looser or no criteria for matching in other parts of the metadata. For example, a perfect match for co-morbidities and sex may be required, age may need to match within a certain threshold, and there may be no requirement for matching of ethnicity.

Of course, the predefined set of criteria may be based on user preferences indicating target matching characteristics. Thus, a user may prescribe what characteristics (described by the metadata) of the historic subjects need to match the query subject in order for the historic subject to be considered a candidate subject. This may be particularly useful when a clinician believes a certain characteristic of the query subject is highly relevant to the diagnosis and treatment of the subject.

In step 134, the vector embeddings of each candidate subject are compared. The comparison may involve determining a proximity in space between the vector embeddings, and a ranking based on the determined proximity.

In some embodiments, the comparison 134 of the vector embeddings includes generating, for each vector embedding of the identified candidate subject entries, a similarity score indicating a degree of similarity between the query vector embedding and the respective vector embedding of the identified candidate subject entry. This similarity score may then be used for subsequent identification of the closest matching candidate subject entries.

Furthermore, the comparison may involve processing each of the vector embeddings of the identified candidate subject entries and the query vector embedding with a hierarchical navigable small world (HNSW) algorithm. The HNSW algorithm finds the approximate nearest neighbors to the query vector embedding amongst the vector embeddings of the identified candidate subject entries approximate nearest neighbors of the query candidate. The nearest neighbors may then be determined based on a variety of similarity metrics, such as Euclidian distance, cosine similarity, and so on. Thus, this provides a quantifiable way of comparing a difference between each of the identified candidate subject entries with the query vector embedding. Nonetheless, other algorithms suitable for comparison of vectors are well known and would be readily implemented by the skilled person.

In additional embodiments of the invention, step 130 may comprise encoding the metadata with the ECG data, thus providing a unified vector embedding. That is, the query vector embedding and the query metadata are combined to provide a unified query vector embedding. The unified query vector embedding may then be compared to unified recorded vector embeddings (each unified recorded vector embedding being a combination of the recorded metadata and recorded vector embedding of the respective subject entry). Thus, the unified vector embeddings may be compared similarly to the way in which the vector embeddings of the ECG data are compared as described above. In this case, the filtering step 132 may be skipped, with the comparison providing for the matching of the query subject to subject entries based on the ECG data and metadata. In step 140, similar subject entries are identified based on the result of the comparison. As a result, subject entries amongst the plurality of subject entries in the database that most closely match the query subject are identified.

The identification 140 of the one or more similar subject entries may be based on the generated similarity scores described above. In some cases, the method may further involve ranking the identified similar subject entries based on a degree of similarity between the respective recorded vector embeddings of the similar subject entries and the query vector embedding (i.e., based on the similarity scores), and/or the respective recorded metadata of the similar subject entries and the query metadata (i.e., based on a score reflecting the degree to which the metadata matches).

Finally, in optional step 150, at least part of the recorded metadata, the ECG data, reported clinical findings and/or subject outcomes associated with the identified similar subject entries are provided. Thus, this information can be presented to a clinician to aid in decision making.

This information may be provided on a user interface and may be interactable for a clinician to be presented with information of all other matching data. For example, the matched subjects and their respective treatment pathway and clinical outcomes may be presented to a clinician. In another example in which four similar subject entries are identified, each of these matched subjects may be presented in a list (which may be ranked based on similarity to the query subject), along with at least part of their metadata, reported findings, outcomes, and a severity of the case. More information may be available should the user indicate that this is desired.

Of course, this information may be presented to the clinician in a user-friendly format, such as a graphical user interface, to aid in decision making. In some cases, the severity reported for the matching cases can help with subject triage. For example, if the top two matches both have a severity category of emergency, this may suggest a high likelihood that the query subject might require immediate attention.

To summarize, the flowchart 100 highlights the sequence of steps for processing and analyzing medical data to find similar cases. The method incorporates both metadata comparison and vector embedding analysis to enhance the accuracy of identifying similar subject entries. This approach allows for efficient searching and matching of complex medical data, potentially improving clinical decision-making processes.

In order to provide a proof of concept of the invention, an ECG encoder was trained in a self-supervised learning (SSL) fashion and used to generate a vector embedding of ECG data for each of 9000 subject entries. A query subject was then randomly selected from a database of 9000 subject entries. 100 of the subject entries with vector embeddings nearest the vector embedding of the query subject were then identified amongst the 9000 subject entries.

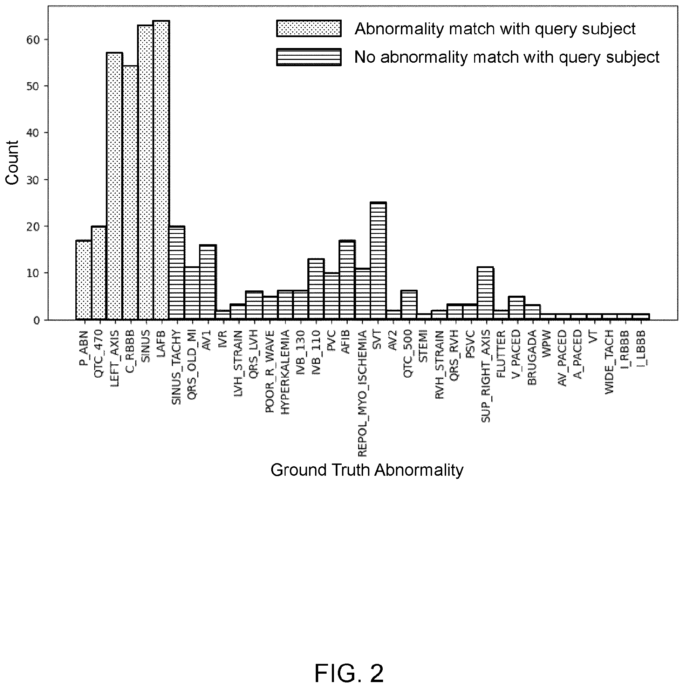

FIG. 2 thus presents a histogram of cardiac abnormalities that were diagnosed for each of these 100 subjects by cardiologists. It is seen that all abnormalities that were diagnosed for the query subject (the matching bars) are also present on most of the subjects that were amongst the 100 closest matches. This therefore shows that it is possible to successfully capture cardiac abnormality information within the vector embeddings created by the ECG encoder. That is, the representation of the ECG data in the form of vector embeddings by the ECG encoder retains key information that can be used to match other ECG data with similar cardiac abnormalities.

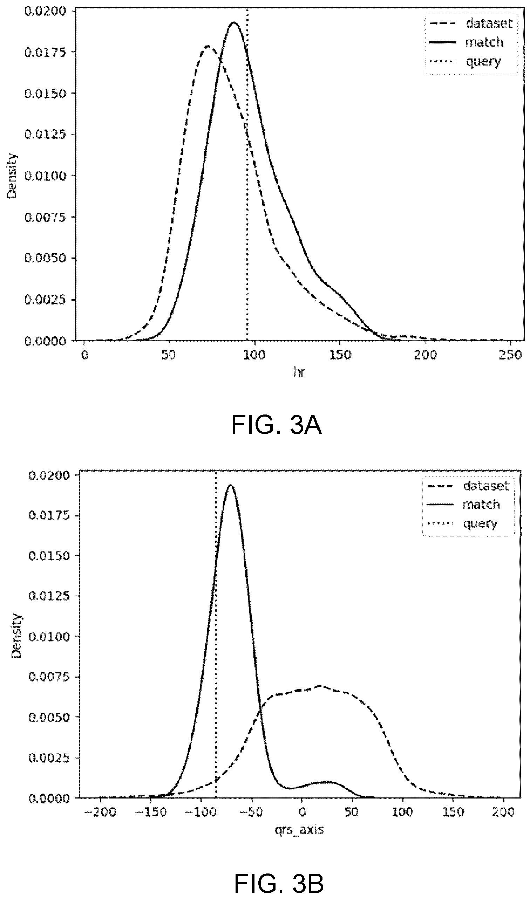

In FIGS. 3A-3C present the distribution of different measurements extracted from the ECG data of each of the 100 closest subjects to the query subject above, the distribution of different measurements extracted from the ECG data of each of the 9000 subjects, and the measurement extracted from the ECG data of the query subject.

Specifically, Fig. 3A presents the heart rate of each of the subjects in the dataset, each of the subjects in the 100 closest subjects, and the query subject. As can be seen, the query subject has a heart rate of 96 BPM, with a distribution peak of the 100 closest subjects much closer to the query subject than the whole dataset.

Fig. 3B presents the QRS axis measurement (in degrees) of each of the subjects in the dataset, each of the subjects in the 100 closest subjects, and the query subject. The query subject has a left axis deviation, whilst the 100 closest subjects also present this in general, with a larger distribution of the whole dataset spread across the normal axis range of -30 to 90 degrees.

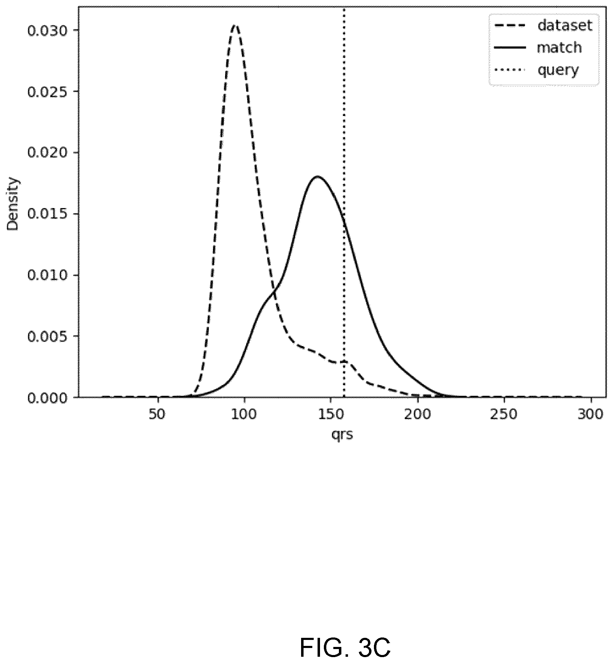

Fig. 3C presents the QRS duration (in milliseconds) of each of the subjects in the dataset, each of the subjects in the 100 closest subjects, and the query subject. The query subject has QRS duration of over 150ms, whilst the 100 closest subjects also present a QRS duration of roughly 150ms on average, whilst the whole dataset has a roughly 100ms QRS duration.

Thus, it can be seen from each of these graphs that the vector embeddings that most closely match the vector embedding of the query subject are associated with subjects that have similar cardiovascular measurements and conditions to the query subject. This shows that the ECG encoder enables accurate and quick matching of the ECG data.

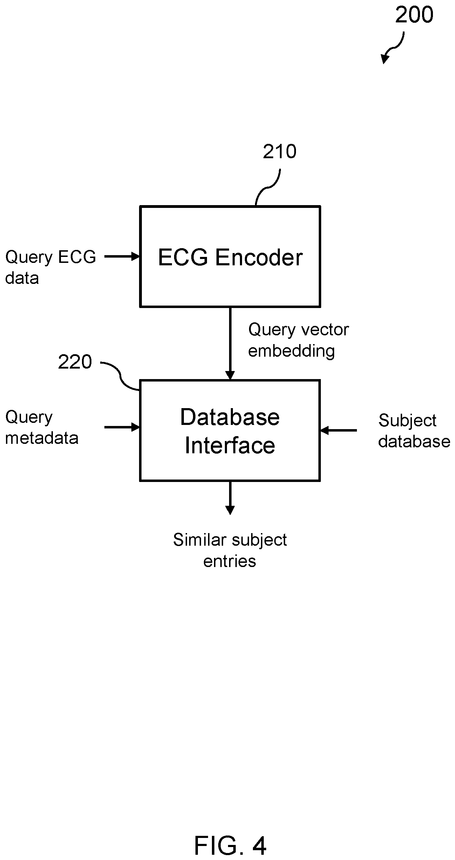

Moving on, FIG. 4 depicts a block diagram of a system for searching a medical database comprising ECG data, in accordance with example embodiments. The system comprises an ECG Encoder 210 and a Database Interface 220.

The ECG Encoder 210 is configured to receive query ECG data as input and generate a query vector embedding. In some aspects, the ECG Encoder 210 may be a neural network that is trained to detect features on an ECG and convert these detected features into a vector. This represents

In some embodiments, the ECG Encoder 210 may be implemented as a vision transformer neural network trained in a self-supervised manner. This type of neural network is designed to detect features on an ECG and convert these detected features into a vector. The vector embedding represents the ECG data in a high-dimensional space that encodes the semantic meaning of the signal. Therefore, signals that are close together in this space are also physiologically closer than the signals that are far apart in this space. This allows for a more nuanced comparison of physiological similarities between subjects, enhancing the precision of the subject matching process.

The Database Interface 220 is configured to receive the query vector embedding from the ECG Encoder 210, along with query metadata from another source (e.g., directly from the subject, a questionnaire, or entered by a clinician). The query metadata may describe characteristics of the query subject, such as age, sex, comorbidities, and other relevant information. The Database Interface 220 is also connected to a medical database, which comprises a plurality of subject entries. Each subject entry corresponds to a historic subject and includes recorded metadata describing characteristics of the respective subject and a recorded vector embedding representing ECG data of the respective subject.

The Database Interface 220 processes these inputs to identify one or more similar subject entries. This process involves comparing the recorded metadata of the plurality of subject entries with the query metadata, and the recorded vector embeddings with the query vector embedding. In some cases, the comparison of the metadata and the vector embeddings may involve filtering the historic subject entries to identify candidate subject entries that have similar metadata to the metadata of the query subject. The vector embeddings of the identified candidate subject entries and the query vector embedding are then compared to identify similar subject entries.

In some embodiments, the Database Interface 220 may utilize a hierarchical navigable small world (HNSW) algorithm for comparing the vector embeddings. The HNSW algorithm is designed to process each of the vector embeddings of the identified candidate subject entries and the query vector embedding to determine their similarity. This algorithm operates at extremely fast speeds, enabling the system to go through the subject's entire database to find the closest matches at sub-second speeds.

In some cases, the comparison of the vector embeddings may include generating a similarity score for each vector embedding of the identified candidate subject entries. The similarity score indicates a degree of similarity between the query vector embedding and the respective vector embedding of the identified candidate subject entry. This score may be generated based on various factors, such as the distance between the vector embeddings in the high-dimensional space, the angle between the vector embeddings, or other measures of similarity.

Nevertheless, the system may also utilize other advanced techniques for processing and comparing vector embeddings, such as other types of approximate nearest neighbor search algorithms, other types of similarity score generation methods, or other types of ranking methods. These variations may be selected based on various factors, such as the specific requirements of the application, the characteristics of the ECG data, the characteristics of the medical database, or other relevant factors.

The Database Interface 220 then identifies similar subject entries based on the result of the comparison. The identification of the one or more similar subject entries is based on the generated similarity scores. In some aspects, the Database Interface 220 may further involve ranking the identified similar subject entries based on a degree of similarity between the respective recorded vector embeddings of the similar subject entries and the query vector embedding, and/or the respective recorded metadata of the similar subject entries and the query metadata.

The Database Interface 220 is configured to output the identified similar subject entries. This output may be presented to a clinician to aid in decision making. In some cases, the output may include a summary of the clinical findings and outcomes for each of the identified similar subject entries. This may allow the clinician to review the treatment pathways and outcomes of subjects with similar physiological characteristics and make informed decisions regarding the care pathway for the query subject. In some aspects, the output may also include the ECG data of the identified similar subject entries, allowing the clinician to visually compare the ECG signals of the query subject and the similar subject entries.

The system as described in FIG. 4 enables comparison and retrieval of similar subject records based on both ECG signal characteristics and metadata information. This configuration allows for efficient processing of ECG data and metadata to identify similar subjects in the database. In some aspects, the system may be implemented as part of a larger health IT infrastructure, which may include other components and data modalities.

While the system has been described primarily in the context of ECG data, it may also be adapted to work with other types of clinical data. In some aspects, the system may be extended to use imaging data, such as magnetic resonance imaging (MRI), computed tomography (CT), ultrasounds, and x-rays, for subject matching. Indeed, the invention may be applied to any high dimension data (e.g., radiation dose volumes generated from treatment planning systems for radiation therapy). In other cases, the system may use lab test reports, photoplethysmography (PPG) signals, pathology slides, and clinician notes for subject matching. This flexibility allows for a more holistic approach to subject matching and clinical decision support, further enhancing the potential for precision medicine.

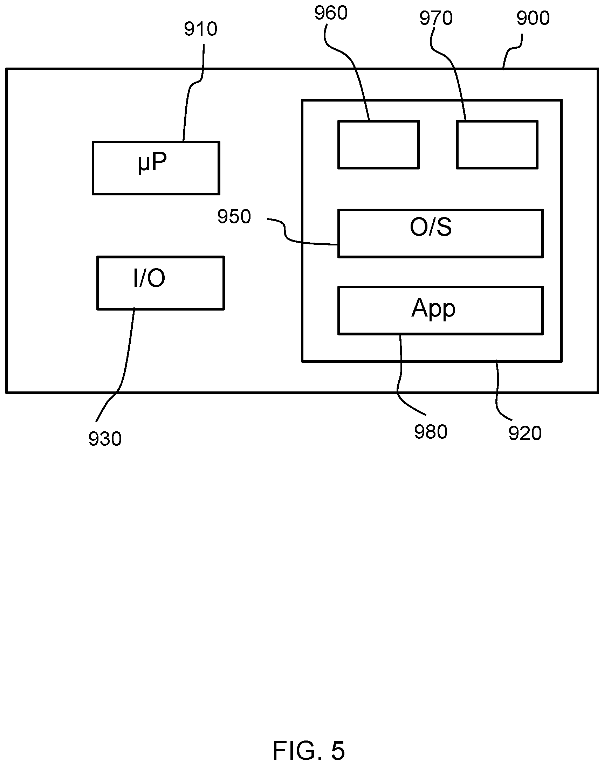

FIG. 5 illustrates an example of a computer 900 within which one or more parts of an embodiment may be employed. Various operations discussed above may utilize the capabilities of the computer. For example, one or more parts of a proposed embodiment may be incorporated in any element, module, application, and/or component discussed herein. In this regard, it is to be understood that system functional blocks can run on a single computer or may be distributed over several computers and locations (e.g. connected via internet), such as a cloud-based computing infrastructure.

The computer 900 includes, but is not limited to, PCs, workstations, laptops, PDAs, palm devices, servers, storages, and the like. Generally, in terms of hardware architecture, the computer 900 may include one or more processors 910, memory 920 and one or more I/O devices 930 that are communicatively coupled via a local interface (not shown). The local interface can be, for example but not limited to, one or more buses or other wired or wireless connections, as is known in the art. The local interface may have additional elements, such as controllers, buffers (caches), drivers, repeaters, and receivers, to enable communications. Further, the local interface may include address, control, and/or data connections to enable appropriate communications among the aforementioned components.

The processor 910 is a hardware device for executing software that can be stored in the memory 920. The processor 910 can be virtually any custom made or commercially available processor, a central processing unit (CPU), a digital signal processor (DSP), or an auxiliary processor among several processors associated with the computer 900, and the processor 910 may be a semiconductor-based microprocessor (in the form of a microchip) or a microprocessor.

The memory 920 can include any one or combination of volatile memory elements (e.g., random access memory (RAM), such as dynamic random access memory (DRAM), static random access memory (SRAM), etc.) and non-volatile memory elements (e.g., ROM, erasable programmable read only memory (EPROM), electronically erasable programmable read only memory (EEPROM), programmable read only memory (PROM), tape, compact disc read only memory (CD-ROM), disk, diskette, cartridge, cassette or the like, etc.). Moreover, the memory 920 may incorporate electronic, magnetic, optical, and/or other types of storage media. Note that the memory 920 can have a distributed architecture, where various components are situated remote from one another, but can be accessed by the processor 910.

The software in the memory 920 may include one or more separate programs, each of which comprises an ordered listing of executable instructions for implementing logical functions. The software in the memory 920 includes a suitable operating system (O/S) 950, compiler 960, source code 970, and one or more applications 980 in accordance with exemplary embodiments. As illustrated, the application 980 comprises numerous functional components for implementing the features and operations of the exemplary embodiments. The application 980 of the computer 900 may represent various applications, computational units, logic, functional units, processes, operations, virtual entities, and/or modules in accordance with exemplary embodiments, but the application 980 is not meant to be a limitation.

The operating system 950 controls the execution of other computer programs, and provides scheduling, input-output control, file and data management, memory management, and communication control and related services. It is contemplated by the inventors that the application 980 for implementing exemplary embodiments may be applicable on all commercially available operating systems.

Application 980 may be a source program, executable program (object code), script, or any other entity comprising a set of instructions to be performed. When a source program, then the program is usually translated via a compiler (such as the compiler 960), assembler, interpreter, or the like, which may or may not be included within the memory 920, so as to operate properly in connection with the O/S 950. Furthermore, the application 980 can be written as an object oriented programming language, which has classes of data and methods, or a procedure programming language, which has routines, subroutines, and/or functions, for example but not limited to, C, C++, C#, Pascal, Python, BASIC, API calls, HTML, XHTML, XML, ASP scripts, JavaScript, FORTRAN, COBOL, Perl, Java, ADA, .NET, and the like.

The I/O devices 930 may include input devices such as, for example but not limited to, a mouse, keyboard, scanner, microphone, camera, etc. Furthermore, the I/O devices 930 may also include output devices, for example but not limited to a printer, display, etc. Finally, the I/O devices 930 may further include devices that communicate both inputs and outputs, for instance but not limited to, a NIC or modulator/demodulator (for accessing remote devices, other files, devices, systems, or a network), a radio frequency (RF) or other transceiver, a telephonic interface, a bridge, a router, etc. The I/O devices 630 also include components for communicating over various networks, such as the Internet or intranet.

If the computer 900 is a PC, workstation, intelligent device or the like, the software in the memory 920 may further include a basic input output system (BIOS) (omitted for simplicity). The BIOS is a set of essential software routines that initialize and test hardware at start-up, start the O/S 950, and support the transfer of data among the hardware devices. The BIOS is stored in some type of read-only-memory, such as ROM, PROM, EPROM, EEPROM or the like, so that the BIOS can be executed when the computer 900 is activated.

When the computer 900 is in operation, the processor 910 is configured to execute software stored within the memory 920, to communicate data to and from the memory 920, and to generally control operations of the computer 900 pursuant to the software. The application 980 and the O/S 950 are read, in whole or in part, by the processor 910, perhaps buffered within the processor 910, and then executed.

When the application 980 is implemented in software it should be noted that the application 980 can be stored on virtually any computer readable medium for use by or in connection with any computer related system or method. In the context of this document, a computer readable medium may be an electronic, magnetic, optical, or other physical device or means that can contain or store a computer program for use by or in connection with a computer related system or method.

The application 980 can be embodied in any computer-readable medium for use by or in connection with an instruction execution system, apparatus, or device, such as a computer-based system, processor-containing system, or other system that can fetch the instructions from the instruction execution system, apparatus, or device and execute the instructions. In the context of this document, a "computer-readable medium" can be any means that can store, communicate, propagate, or transport the program for use by or in connection with the instruction execution system, apparatus, or device. The computer readable medium can be, for example but not limited to, an electronic, magnetic, optical, electromagnetic, infrared, or semiconductor system, apparatus, device, or propagation medium.

The proposed method(s), device(s) and/or system(s) may be implemented in hardware or software, or a mixture of both (for example, as firmware running on a hardware device). To the extent that an embodiment is implemented partly or wholly in software, the functional steps illustrated in the process flow diagrams may be performed by suitably programmed physical computing devices, such as one or more central processing units (CPUs) or graphics processing units (GPUs). Each process - and its individual component steps as illustrated in the flow diagrams - may be performed by the same or different computing devices. According to embodiments, a computer-readable storage medium stores a computer program comprising computer program code configured to cause one or more physical computing devices to carry out a control method as described above when the program is run on the one or more physical computing devices.

Storage media may include volatile and non-volatile computer memory such as RAM, PROM, EPROM, and EEPROM, optical discs (like CD, DVD, BD), magnetic storage media (like hard discs and tapes). Various storage media may be fixed within a computing device or may be transportable, such that the one or more programs stored thereon can be loaded into a processor.

To the extent that an embodiment is implemented partly or wholly in hardware, some of the blocks shown in the block diagrams may be separate physical components, or logical subdivisions of single physical components, or may be all implemented in an integrated manner in one physical component. The functions of one block shown in the drawings may be divided between multiple components in an implementation, or the functions of multiple blocks shown in the drawings may be combined in single components in an implementation. Hardware components suitable for use in embodiments of the present invention include, but are not limited to, conventional microprocessors, application specific integrated circuits (ASICs), and field-programmable gate arrays (FPGAs). One or more blocks may be implemented as a combination of dedicated hardware to perform some functions and one or more programmed microprocessors and associated circuitry to perform other functions.

Variations to the disclosed embodiments can be understood and effected by those skilled in the art in practicing the claimed invention, from a study of the drawings, the disclosure, and the appended claims. In the claims, the word "comprising" does not exclude other elements or steps, and the indefinite article "a" or "an" does not exclude a plurality. A single processor or other unit may fulfil the functions of several items recited in the claims. The mere fact that certain measures are recited in mutually different dependent claims does not indicate that a combination of these measures cannot be used to advantage. If a computer program is discussed above, it may be stored/distributed on a suitable medium, such as an optical storage medium or a solid-state medium supplied together with or as part of other hardware, but may also be distributed in other forms, such as via the Internet or other wired or wireless telecommunication systems. If the term "adapted to" is used in the claims or description, it is noted the term "adapted to" is intended to be equivalent to the term "configured to". Any reference signs in the claims should not be construed as limiting the scope.

The flow diagrams and block diagrams in the Figures illustrate the architecture, functionality, and operation of possible implementations of systems, methods, and computer program products according to various embodiments of the present invention. In this regard, each block in the flow diagrams or block diagrams may represent a module, segment, or portion of instructions, which comprises one or more executable instructions for implementing the specified logical function(s). In some alternative implementations, the functions noted in the block may occur out of the order noted in the Figures. For example, two blocks shown in succession may, in fact, be executed substantially concurrently, or the blocks may sometimes be executed in the reverse order, depending upon the functionality involved. It will also be noted that each block of the block diagrams and/or flow diagrams and combinations of blocks in the block diagrams and/or flow diagrams, can be implemented by special purpose hardware-based systems that perform the specified functions or acts or carry out combinations of special purpose hardware and computer instructions.

## Claims

1. A method for searching a medical database comprising electrocardiogram, ECG data, the method comprising:
obtaining (110) a database comprising a plurality of subject entries each corresponding to a historic subject, each subject entry comprising recorded metadata describing characteristics of the respective subject and a recorded vector embedding representing ECG data of the respective subject;
generating (120), with an ECG encoder, a query vector embedding representing ECG data of a query subject;
comparing (130) the recorded metadata of the plurality of subject entries with query metadata describing characteristics of the query subject, and the recorded vector embeddings with the query vector embedding; and
identifying (140) one or more similar subject entries based on a result of the comparison.

2. The method of claim 1, wherein comparing (130) the metadata and the vector embeddings, comprises:
filtering (132) the historic subject entries to identify candidate subject entries, the candidate subject entries comprising similar metadata to the metadata of the query subject; and
comparing (134) the vector embeddings of the identified candidate subject entries and the query vector embedding.

3. The method of claim 2, wherein comparing (134) the vector embeddings comprises processing, with a hierarchical navigable small world, HNSW, algorithm, each of the vector embeddings of the identified candidate subject entries and the query vector embedding.

4. The method of claim 2 or 3, wherein comparing (134) the vector embeddings comprises generating, for each vector embeddings of the identified candidate subject entries, a similarity score indicating a degree of similarity between the query vector embedding and the respective vector embedding of the identified candidate subject entry, and wherein
identifying (140) the one or more similar subject entries is based on the generated similarity scores.

5. The method of any of claims 2-4, wherein filtering (132) the historic subject entries is based on a predefined set of criteria defining matching characteristics between the metadata of the subject entry and the metadata of the query subject.

6. The method of claim 5, wherein the predefined set of criteria is based on user preferences indicating target matching characteristics.

7. The method of any of claims 1-6, wherein the ECG encoder is a neural network adapted to detect features of ECG data and convert the detected features into a vector.

8. The method of claim 7, wherein the neural network is a vision transformer.

9. The method of any of claims 1-8, further comprising providing (150) at least part of the recorded metadata, the ECG data, reported clinical findings and/or subject outcomes associated with the identified similar subject entries.

10. The method of any of claims 1-9, further comprising ranking the identified similar subject entries based on a degree of similarity between the respective recorded vector embeddings of the similar subject entries and the query vector embedding, and/or the respective recorded metadata of the similar subject entries and the query metadata.

11. The method of any of claims 1-10, wherein the metadata comprises subject demographic information, subject medical information, subject condition information, and subject context information.

12. The method of any of claims 1-11, wherein the vector embeddings are a projection of the ECG data in high dimension space that encodes semantic meaning of the ECG data.

13. A computer program comprising computer program code means adapted, when said computer program is run on a computer, to implement the method of any of claims 1-12.

14. A system for searching a medical database, the medical database comprising a plurality of subject entries each corresponding to a historic subject, each subject entry comprising recorded metadata describing characteristics of the respective subject and a recorded vector embedding representing ECG data of the respective subject, the system comprising:
an ECG encoder (210) configured to generate a query vector embedding representing ECG data of a query subject;
a database interface (220) configured to:
compare the recorded metadata of the plurality of subject entries with query metadata describing characteristics of the query subject, and the recorded vector embeddings with the query vector embedding; and
identify one or more similar subject entries based on a result of the comparison.

15. The system of claim 14, wherein the ECG encoder (210) is a neural network adapted to detect features of ECG data and convert the detected features into a vector.
